Europäisches Patentamt

European Patent Office

Office européen des brevets

(11) Publication number : **0 576 279 A1**

(12) **EUROPEAN PATENT APPLICATION**

(21) Application number : 93304941.3

(22) Date of filing : 24.06.93

(51) Int. Cl.⁵ : **A61K 47/36**

(30) Priority : **26.06.92 US 904783**

(43) Date of publication of application :
**29.12.93 Bulletin 93/52**

(84) Designated Contracting States :
**AT BE CH DE DK ES FR GB GR IE IT LI LU MC NL PT SE**

(71) Applicant : **GENZYME CORPORATION**
**One Kendall Square**
**Cambridge, Massachusetts 02139-1562 (US)**

(72) Inventor : **Dow, Gordon J.**
**3868 Lanewood Way**
**Santa Rosa, California 95404 (US)**
Inventor : **Rasmussen, James R.**
**75-83 Cambridge Parkway**
**E-411 Cambridge, Massachusetts 02142 (US)**

(74) Representative : **Froud, Clive et al**
**Elkington and Fife Prospect House 8**
**Pembroke Road**
**Sevenoaks, Kent TN13 1XR (GB)**

(54) Hydrophilic cream for the delivery of therapeutic agents.

(57) Inter alia, a hydrophilic cream for application to an open wound or a mucous membrane characterised in that it comprises an aqueous vehicle, a therapeutic agent selected from debriding enzymes, wound healing agents and antibacterial agents, and a non-pyrogenic seed galactomannan thickening agent is disclosed.

EP 0 576 279 A1

This invention relates to a sterile hydrophilic cream thickened with an appropriate thickening agent that is designed to deliver both neutral and charged therapeutic agents to open wounds and mucous membranes, and to maintain those agents on the treatment site, even in the presence of exudate.

Open wounds, such as burns, ulcers and surgical incisions provide a portal to the systemic system for contaminates, including toxins, immunogens and pyrogens. In the case of topical therapeutics for burn treatment where extensive body surface area may be involved, large quantities of a topical agent may be required to be applied to a patient. For example, application of a topical cream at 1.0 mm thickness to 50% of the total body surface area of a 70 Kg burn patient would require approximately 850 g of that cream. Thus, a 1% contaminant in the topical agent would result in the application of 8.5 g of the contaminant to the patient.

Many topical therapeutic agents, such as debriding enzymes and wound healing factors, are peptides or proteins that have some inherent charge at the physiological pH. As with ion exchange chromatographic resins, a charged molecule may bind to an oppositely charged polymeric thickening agent by ionic interactions. The tight association of the therapeutic agent to a high molecular weight polymeric thickener may inhibit diffusion of the therapeutic agent to the wound site and may impair the delivery or action of the therapeutic agent at the wound site. Thus, an appropriate thickening agent is one that is uncharged in aqueous solution at the physiological pH, and is substantially free of contaminating substances.

Galactomannans are relatively widespread in nature having been identified in the cell walls and endosperm of certain plant seeds and in the mycelia of various fungi. The galactomannas which have been found particularly useful in the practice of this invention are the seed galactomannans. Seed galactomannans are polysaccharides that are derived from botanical sources and have the characteristics as stated in A.M. Stephen, The Polysaccharides, pages 115-116 (1983). Two of the more commonly used seed galactomannans are derived from guar gum and locust bean gum. The distinguishing feature between these two seed-derived galactomannans is their compositional ratio of mannose to galactose; guaran has a mannose:galactose ratio of about 2:1 and locust bean gum has a ratio of about 4:1. Neither of these polysaccharides have ionizable groups and, thus, remain uncharged in aqueous solution.

Guaran has a high viscosity when dissolved in aqueous solution, and the crude form of guaran, guar gum, has been previously used as a thickening agent in foods, lotions, and creams and as a binder in tablets. Guaran's viscoelastic properties make it an attractive thickening ingredient for drug delivery vehicles to open wounds such as ulcers, surgical wounds and the eschar of burn victims, and to mucous membranes. Because the potential exists for the guaran to enter the circulatory system of these patients, it is desirable to have a guaran preparation with low contaminant levels, especially low protein and low endotoxin content. The present invention describes an ultrapure preparation of guaran and a method for preparing it from guar gum.

One indication for use of a viscous, sterile hydrophilic cream is the delivery of therapeutic agents, such as debriding enzymes, to burn wounds. Enzymatic debridement of burn eschar is an attractive alternative to surgical excision as it minimizes blood loss and surgical trauma and, through biological specificity, may distinguish between necrotic and viable tissue. Because the major constituents of burn eschar are denatured proteins, a variety of proteases have been explored as debriding agents. Ananain and comosain have been identified as two major protease components of crude bromelain and are more fully described in commonly assigned US Patent No. 5,106,621, issued April 21, 1992, the pertinent disclosure of which is incorporated herein by reference. These two proteases constitute approximately 5% of the soluble protein in crude bromelain, and may be chromatographically purified from pineapple stem powder to yield a well defined preparation. A successful vehicle for the delivery of an enzymatic debriding agent must be safe, efficient at enzyme delivery, sufficiently viscid to maintain the proteolytic enzymes on the wound site, hydrophilic to hydrate the wound bed, capable of absorbing wound exudate, and easy to apply to and cleanse from the wound site. Attempts to formulate the ananain and comosain proteases in an effective delivery vehicle have proven challenging for two reasons. First, many known topical creams and gels soften dramatically when applied to a wound and will migrate from a wound site when diluted with wound fluid. Secondly, the ananain and comosain cysteine proteases are strongly cationic with pI >8.5 and may bind by ionic interactions to commonly used anionic thickening agents, such as polysaccharides and synthetic gums, when formulated at physiological pH.

It is therefore an objective of this invention to provide a suitable thickening agent for the efficient delivery of therapeutic agents to a wound site. The cream should have properties such that it is not so thick and stiff that it is difficult to apply to the application site, yet it maintains its viscosity as it is diluted with bodily exudate.

In accordance with the present invention, a viscous hydrophilic cream for application to an open wound comprises a therapeutic agent selected from the group consisting of debriding enzymes, wound healing agents and anti bacterial agents, and a substantially uncharged, pyrogen-free seed galactomannan, preferably derived from guar gum or locust bean gum.

For the debridement of necrotic tissue resulting from thermal injury or an ulcer, the therapeutic agent may comprise one or more cysteine proteases. Preferably, the cysteine proteases are ananain and comosain derived from pineapple stem powder, and the galactomannan thickening agent is guaran that has been purified to reduce its protein and endotoxin levels.

The hydrophilic cream formulated with the therapeutic agent is typically applied topically to the necrotic tissue, such as burn eschar, and the debriding proteases are allowed to diffuse from the vehicle to the tissue. After a suitable period of exposure, that may encompass more than one application over several hours, the site is cleansed to remove the cream.

The hydrophilic vehicle prepared in accordance with this invention preferably consists of an oil-in-water cream base thickened with a high purity seed galactomannan, preferably locust bean gum or guaran. The debriding agent prepared in accordance with this invention consists of two pineapple stem-derived cationic cysteine proteases, ananain and comosain, in a guaran-thickened hydrophilic cream vehicle. Additional ingredients may include enzyme activators and preservatives to inhibit bacterial growth.

The hydrophilic cream vehicle consists of two key components: 1) an oil-in-water cream base, and 2) high purity guaran or locust bean gum.

The hydrophilic cream base may have the following components with the following approximate nominal and preferred concentration ranges (given in percent by weight) :

| Ingredient | Nominal | Preferred |
|---|---|---|
| Oleagenous material(s) | | |
| (Oil Phase) | 0.5 - 60 | 5 - 40 |
| Emulsifier(s) | 0.2 - 10 | 1 - 5 |
| Antimicrobial | | |
| Preservative(s) | 0.05 - 2.5 | 0.2 - 1.5 |
| Additives | 0 - 20 | 0 - 5 |
| Purified Water | QSAD* 100 | QSAD 100 |

*QSAD = Sufficient quantity to make

Additives may also include buffers (such as sodium phosphate), humectants (such as glycerin or sorbitol) and other excipients known to those skilled in the art.

Guaran is a galactomannan extracted from the endosperm of guar seeds (Cyamopsis tetragonolobus). Food-grade guar gum is commercially available from a number of companies including Aqualon Company (Wilmington, DE) and Hi-Tek Polymers Inc. (Clifton, NJ). The technical specifications for food-grade guar gum allow an impurity content of at least 4% protein. The impure product is not sold in sterile form, but it frequently contains a low bioburden with microbial limits being less than 500 colony forming units/g of guar gum. It has been found that food-grade guar gum is pyrogenic in the rabbit pyrogen test (USP XXII, p. 1515) and elicits a high response of about 5 endotoxin units per mg in the Limulus Amoebocyte Lysate test (USP XXII, p. 1493).

Ultrapure guaran may be prepared by a combination of filtration and solvent precipitation steps. Food-grade guar gum, made by procedures described in the literature (see F. Smith and R. Montgomery, The Chemistry of Plant Gums and Mucilages (1959)), or purchased from commercial suppliers (Aqualon Co., Wilmington, DE), is suitable as a starting material in this invention. The gum is suspended in an aqueous solution at a concentration of 0.25% to 0.5%, preferably at a concentration of 0.25%. The aqueous solution may contain salts (for example, sodium chloride or sodium acetate) and/or buffers (for example, Tris or acetate). The pH of the solution may be between pH 3 and pH 9, and preferably about pH 6.5. If desired, an antimicrobial agent such as methyl paraben may be added. Dissolution can take place between 4°C and 80°C and may be encouraged by gentle stirring or shaking of the solution. Dissolution is considered to be complete when the viscosity of the solution does not increase over a 1 hour period. Following dissolution, the solution may be subjected to centrifugation or filtered to remove insolubles with the preferred step being filtration. A number of commercial filters may be employed. For example filtration through a NA 1000 filter cartridge (supplier FPI Inc., Sacramento, CA) removes large insoluble particles. Further purification may be achieved by additional filtration steps and solvent precipitation. For example, sequential filtration through a NA 600 filter cartridge, NA 45 filter cartridge, and a 0.2 μ Posidyne filter cartridge (Pall; Glen Core, NY), produces a clear solution suitable for the subsequent precipitation step. Other combinations of filters are possible. The majority of pyrogenic material is removed by the filtration steps.

Precipitation of the guaran from the filtered solution is accomplished by mixing with a water miscible solvent such as a $C_1$-$C_4$ alcohol or acetone. Preferred solvents include ethanol or isopropyl alcohol. Either addition of the guaran solution to the organic solvent or addition of the solvent to the guaran solution leads to satisfactory precipitation, with the latter being preferred. If ethanol is used as a solvent, a 1.5:1 volumetric ratio of ethanol to guaran solution gives a satisfactory precipitate. Much of the guaran precipitates as fibers. The guaran is collected and washed with 95% aqueous ethanol and then dried by freeze drying or vacuum drying.

Guaran prepared by this method shows markedly reduced levels of protein (<0.2% w/w) and endotoxin (<0.03 endotoxin units/mg; USP XXII, p. 1493) and is not pyrogenic when administered systemically to rabbits at 33 mg/Kg (USP XXII, p 1515). In addition, the seed galactomannan retains its viscoelastic properties and a high molecular weight. The terms "ultrapure" and "pyrogen free" thus define a seed galactomannan having these general characteristics.

A hydrophilic cream vehicle, such as an oil-in-water emulsion, thickened with high purity guaran may be formulated by separate preparation of the aqueous and oil phase components followed by admixing at 60-70 °C. The aqueous phase may contain a preservative and the seed galactomannan thickening agent, and optionally a buffering agent. The oil phase contains the water insoluble components. To maximize the hydrophilic nature of the vehicle, the oil phase is preferably minimized to be less than about 40% by weight, preferably between about 5% and 30% by weight. Suitable oil-in-water hydrophilic cream vehicles for purposes of this invention, for example, are described in U.S. Patent 3,761,590, issued September 25, 1973 to Fox, and in Formula A & B as follows:

| FORMULA A | | Percent by Weight |
|---|---|---|
| Cethyl Alcohol | | 12 |
| White Wax | | 1 |
| Mineral Oil | | 3 |
| Propylene Glycol | | 5 |
| Imidurea | | 0.3 |
| Polyoxyethylene | | |
| stearyl ether (Brij 72) | | 0.6 |
| Polyoxyethylene | | |
| stearyl ether (Brij 721) | | 4.0 |
| Galactomannan | | 2.5 |
| Purified Water | QSAD | 100.0 |

| FORMULA B | Percent by Weight |
|---|---|
| White Petrolatum | 7.0 |
| Isostearyl Alcohol | 5.0 |
| Glyceryl Monostearate | 5.0 |
| Cetyl Alcohol | 1.5 |
| Sorbitol Solution USP | 5.0 |
| Benzyl Alcohol | 1.5 |
| Polyoxyl 40 Stearate | 3.5 |
| Polyoxyethyl (4) Lauryl Ether | 1.0 |
| Galactomannan | 2.5 |
| Purified Water          QSAD | 100.0 |

Any creams of the oil-in-water emulsion type are suitable for the purposes of this invention. However, the most preferred will be free of ionic materials that may interact with the ananain and comosain enzymes, or other charged therapeutic agents. The preferred creams will have oil phases of less than about 40% by weight, and most preferably between about 5 and 30% by weight. Various ingredients known to those skilled in the art may be included in the hydrophilic cream vehicle including but not limited to antimicrobial preservatives, antioxidants, drug stabilizers, activators, and buffers.

The amount of seed galactomannan contained in the vehicle is generally in the range of from about 0.2% to about 7% and preferably from about 2% to about 4%.

The vehicle may be formulated routinely on a 1 Kg scale in glass breakers. A laboratory mixer fitted with a static head homogenizer or a high speed propeller mixer may be used for suspending the guaran. Preferably, the guaran is added at room temperature rapidly so that a uniform suspension may be achieved before the viscosity of the aqueous phase increases to a point where dispersion of the guaran is no longer mechanically possible. Mixing of the the hot oil phase into the hot aqueous phase is accomplished with a standard laboratory high speed mixer. Alternatively, relatively high amounts of guaran can be incorporated homogeneously into the hydrophilic cream vehicle during manufacture. This is accomplished by rapid addition of the guaran to the hot emulsion, immediately after the oil phase has been added to the water phase, with continued rapid agitation.

The formulated vehicle may be thermally sterilized batchwise and delivered to sterile vials aseptically, or the vehicle may be aliquoted into suitable jars and terminally sterilized, for example by autoclaving. Following sterilization, the vehicle is mixed to maintain a dispersion of the oil phase in the aqueous base until the temperature is <70 °C. In the case of terminal sterilization in bottles, the bottle may be shaken during cooling to maintain the necessary dispersion.

The cationic cysteine proteases, ananain and comosain, are the preferred debriding enzymes of this invention. As used herein, the term "debriding enzyme" means an enzyme which is capable of removing necrotic tissue from burns and dermal ulcers. Ananain and comosain co-purified from pineapple stem powder by a purification scheme that includes solvent extraction, two sequential ion exchange chromatography steps and diafiltration. The second ion exchange step may be replaced with chromatography on a reversed phase resin. However, ion exchange chromatography is the preferred method. Extraction of the pineapple stem powder may be carried out with an aqueous buffer at pH 4.0-9.0, with the preferred buffer being sodium acetate at pH 5.0. Ion exchange chromatography may be carried out using a variety of cation exchange resins including Poly-Sulfoethyl aspartamide (PolyLC, Columbia, MD) and S-Sepharose (Pharmacia, Piscataway, NJ), with the preferred resin being S-Sepharose. Purification on the first S-Sepharose column is achieved at pH 5.0 by eluting contaminants at a low buffer/salt concentration of <0.6 M sodium acetate, and elution of the partially purified ananain and comosain containing fraction with high buffer/salt concentration. Purification on the second S-Sepharose column may be achieved by a combination of ionic strength effects and pH changes. Residual contaminants with low binding affinity may be eluted at low buffer/salt concentration, and additional contaminants may be eluted at elevated pH with the preferred pH being 9.0 in an aqueous sodium carbonate buffer. The co-purified ananain and comosain may be eluted from the second cation exchange column by increasing the salt concentration at pH 9.0, or at high buffer salt concentration at pH 5.0. The preferred elution buffer for the second ion exchange chromatographic step is 1.6 M sodium acetate at pH 5.0. Alternatively, the second ion exchange step may be replaced with reversed phase chromatography with the preferred chromatography matrix being polystyrene divinylbenzene (PerSeptive Biosystems, Cambridge, MA). The enzymes may be applied to the reversed phase chromatographic system in high buffer/salt concentration at pH 5.0-9.0 and eluted from the reversed phase resin with an aqueous buffer solution containing a C1-C3 alkanol. The preferred buffer for ananain and comosain co-elution is 36% ethanol in 20 mM sodium acetate, 150 mM NaCl at pH 5.8. The salt concentration of the co-purified ananain and comosain may be reduced by a number of methods that include dialysis, gel filtration chromatography or diafiltration with the preferred method being diafiltration. Additionally, if an alkanol is used as a cosolvent, the alkanol may be removed by similar dialysis, gel filtration or diafiltration methods. The purified diafiltered enzyme mixture may be concentrated by ultrafiltration using a YM-10 membrane (Amicon, Danvers, MA) with a molecular weight cutoff of up to 10,000 Daltons.

The purified protease mixture shows only two major bands by Coomassie staining of a 15% SDS-polyacrylamide gel. The protein band that has a mobility corresponding to 23,800 daltons is ananain, and the protein band that has a mobility corresponding to 24,400 daltons is comosain. Typically, the preparation contains an ananain to comosain ratio of about 5.5 to 1. The proteases are isolated by lyophilization and typically formulated in the sterile hydrophilic cream vehicle at 0.5 - 5.0% by weight at the time of use.

Other therapeutic agents which may be usefully employed in the practice of this invention include antibacterial agents, defined as those agents which inhibit bacterial growth or destroy the bacteria, and wound healing agents, defined as growth factors and agents which effect the rate of wound healing.

The effectiveness of a delivery vehicle at releasing an active therapeutic agent to a wound site may be evaluated in vitro by testing the extractability of the therapeutic agent with an appropriate solvent. The preferred extraction solvent for evaluating release of the debriding enzymes ananain and comosain from a vehicle is an aqueous buffer such as 50 mM phosphate, pH 6.5. The extraction may be carried out by any number of mechanical methods that include shaking or mixing of the vehicle and solvent. Following the extraction procedure, insolubles may be removed by centrifugation, and the aqueous phase isolated from an upper oil layer with a pipet. The quantity of therapeutic agent released to the extraction solvent may be evaluated by any appropriate assay. For the release of proteins such as the debriding enzymes ananain and comosain, both the protease activity and the quantity of protein released may be quantified. The activity of ananain and comosain may be evaluated with highly specific commercially available chromogen-producing substrates. Two such substrates, bz-FVR-NHMec and z-RR-NHMec, previously identified for the assay of ananain and comosain, respectively, are described in U.S. Patent No. 5,106,621, issued April 21, 1992. In similar fashion to these coumaride-conjugated peptide substrates (NHMec), the p-nitroanilide-conjugated homologs (pNA), bz-FVR-pNA and z-RR-pNA, show similar specificity with ananain and comosain. The release of protein-based therapeutic agents may be assessed with any standard protein assay with the preferred assay method being the Bradford assay.

The galactomannan-thickened vehicles of this invention were effective at releasing the debriding enzymes ananain and comosain when compared to vehicle formulated without any thickening agent. Vehicles formulated with the anionic polysaccharide thickening agents sodium carboxymethylcellulose and xanthan gum showed less effective delivery.

The effectiveness of the galactomannan-thickened hydrophilic cream vehicle at delivering therapeutic agents to a wound site may be evaluated using an appropriate animal model. To assess the effectiveness of the galactomannan-thickened vehicle at delivering the ananain and comosain debriding enzyme mixture to burn eschar, a full-thickness porcine burn model was used. The therapeutic agent may be formulated in the vehicle at any appropriate concentration. The purified debriding enzymes were formulated in the vehicle at a concentration sufficient to attain complete debridement of desiccated full-thickness wounds over a 10 hour treatment period.

Any appropriate treatment regimen may be followed for delivery of therapeutic agents with the preferred regimen for the debriding enzymes being two, five hour applications over a 10 hour treatment period. The hydrophilic cream formulated with the therapeutic agent may be applied to a wound site at any appropriate thickness with 1.5-2.0 mm thickness being preferred for delivery of the debriding enzymes, ananain and comosain. During the treatment period, the wound site may be covered with an occlusive dressing such as Bioclusive™ (Johnson & Johnson, New Brunswick, NJ) or Tegaderm (3M, St. Paul, MN).

A macroscopic method may be employed to score the effectiveness of an enzymatic debriding agent. For evaluation of full-thickness thermal wound debridement, both the amount of eschar and hair remaining in the wound site may be used as reporters. Results from debridement studies on four pigs showed the galactomannan-thickened vehicle to deliver effectively the ananain and comosain debriding enzymes.

EXAMPLE 1 - Preparation of High Purity Guaran

625 g Guar gum (Aqualon Co., Wilmington, DE) was hydrated in 250 L water for injection (USP) with mixing over 3 hours at 80 °C. The mixture was pumped with a peristaltic pump through NA 600 (5 $\mu$m, 18 sq ft) and NA 45 (0.3 $\mu$m, 18 sq ft) cellulose depth filter cartridges (FPI Inc., Sacramento, CA). The filter cartridges were replaced as they clogged during the process. A total of three NA 600 filter cartridges and two NA 45 cartridges were required to process the entire guaran containing solution. The pyrogenic material was further reduced by filtration of the guaran containing solution through a 0.2 $\mu$m Posidyne filter cartridge (Pall; Glen Core, NY). Approximately six Posidyne membrane cartridges (20 inch) were required to process the entire solution. The guaran was further purified and collected by addition of 1.5 volumes of 95% ethanol to the filtered solution. The precipitated guaran was allowed to settle and washed twice with 30 L of 95% ethanol. The final purified guaran polysaccharide was collected by centrifugation and dried at 40 °C at 100 milliTorr for 24 hours. The guaran purified by this method had the general characteristics described in Table 1.

Table I. Characteristics of Ultrapure Guaran

| Characteristic | Value | Test Method |
|---|---|---|
| Galactomannan Content | >95% | Monosaccharide Analysis |
| Appearance | white fiber or powder | Visual |
| Molecular Weight | 1.0-2.0 x 10$^6$ dalton | SEC MALLS TSK PW4000-PW6000 0.5%solution, pH = 10.0 |
| Protein content | <0.2% | Bradford Micro-assay (BSA Standard) |
| Endotoxin level | <0.03 EU mg $^{-1}$ | USP LAL Test |
| Pyrogenicity | Non-pyrogenic | USP Pyrogen Test (33 mg Kg$^{-1}$) |

EXAMPLE 2 - Preparation of Protease Mixture

The buffers utilized were buffer A: 0.3 M sodium acetate (pH 5.0), 1.0 mM EDTA; buffer B: 0.375 M sodium acetate (pH 5.0), 1.0 mM EDTA; buffer C: 0.6 M sodium acetate (pH 5.0), 1.0 mM EDTA; buffer D: 1.5 M sodium acetate (pH 5.0), 1.0 mM EDTA; buffer E: 50 mM sodium acetate (pH 5.0); buffer F: 63 mM sodium carbonate (pH 9.0), 25 mM sodium chloride; buffer G: 0.5 M sodium acetate (pH 5.0), 1.0 mM EDTA; buffer H: 20 mM sodium acetate, 1.0 mM EDTA.

2 Kg crude pineapple stem powder (Enzyme Development Corportation, New York, New York) was extracted with 20 L buffer A with mixing. After 1 h, the insoluble particulates were removed by a method consisting of centrifugation at 5,900 g for 25 min followed by filtration through a 0.2 $\mu$m nominal filter cartridge (Sartorius, Bohemia, NY). The resulting clear supernatant was applied to a 18 L S-Sepharose column (Pharmacia, Piscataway, NJ) that had been preequilibrated with buffer B. The resin was subsequently washed with 126 L of buffer B and then 90 L buffer C. The cationic cysteine proteases ananain and comosain were co-eluted with 36 L of buffer D. Further purification of this protease fraction was achieved by chromatography on a second S-Sepharose column. The fraction containing ananain and comosain was diluted 4-fold with deionized water and applied to a 3 L S-Sepharose column that had been equilibrated with buffer E. The column was washed sequentially with 6 L of buffer E, 9 L of buffer F, 6 L of buffer E and 18 L of buffer G.

The purified ananain and comosain were eluted with 8 L buffer D. This fraction was diafiltered against buffer H using an Amicon Spiral YM-10 membrane. Diafiltration was completed when the final buffer concentration was 20 mM sodium acetate, 1.0 mM EDTA. The ananain and comosain proteases were subsequently concentrated to 50 mg/mL using the same diafiltration system. The concentrated protease solution was adjusted to 12.5 ±2.5 mM L-cysteine, and the purified proteases isolated by lyophilization.

EXAMPLE 3 - Formulation of Guaran-thickened Hydrophilic Delivery Vehicle

The oil phase was compounded by mixing 100 g white petrolatum (Penreco, Butler, PA), 150 g stearyl alcohol (Sherex Chemical Co., Inc., Dublin, OH), 40 g isopropyl myristate (Inolex Chemical Co., Philadelphia, PA), 2.5 g sorbitan monooleate (ICI Specialty Chemicals, Wilmington, DE), 22.5 g Polyoxyl 40 stearate (ICI Specialty Chemicals, Wilmington, DE) and 2.4 g propyl paraben (Inolex Chemical Co., Philadelphia, PA) in a 2 L beaker that was placed in a 70 °C water bath until all components were melted and mixed until dissolved.

The aqueous phase was compounded by first preparing the vehicle buffer solution. The buffer solution contained 11.34 g sodium phosphate, monobasic monohydrate (Mallinckrodt Specialty Chemicals, Paris, KY), and 1.84 g potassium hydroxide (Mallinckrodt Specialty Chemicals, Paris, KY) in 1305 g water for injection (USP). The pH of the buffer solution was adjusted to pH 6.5 ±0.1 with either potassium hydroxide or sodium phosphate. To 616 g of the buffer solution was added 3.0 g methyl paraben (Inolex Chemical Co., Philadelphia, PA) and 50 g glycerin (Quantum, Emery Div., Cincinatti, OH). After the above materials had been dissolved by mixing, 27.5 g of high purity guaran from Example 1 was added with rapid mixing so that a uniform suspension of the guaran was achieved in less than 5 sec. The compounded aqueous phase was mixed for an additional 10 minutes during which time the viscosity increased.

To mix the two phases, the compounded aqueous phase was first heated to between 65 and 70 °C in a water bath. The compounded 70 °C oil phase was added to the aqueous phase and mixed for 10 minutes.

50 g Quantities of vehicle were dispensed into 100 mL Pyrex glass containers with polypropylene screwcap closures and autoclaved at 121 °C for 15 min on a slow exhaust cycle. As soon as the autoclave chamber reached ambient, the bottles were removed and shaken manually for about 10 sec about every two minutes until the bottles were <50 °C.

EXAMPLES 4 - 7 - Preparation of a Cream Using an Alternative Polysaccharide Thickening Agent

A product was prepared as described in Example 3 except that the high purity guaran was substituted with alternative polysaccharide thickening agents. The thickening agents were formulated at concentrations similar to the high purity guaran that gave a suitably viscous preparation. The following preparations were formulated:

EXAMPLE 4  no thickening agent
EXAMPLE 5  25 g locust bean gum
EXAMPLE 6  27.5 g xanthan gum
EXAMPLE 7  20 g sodium carboxymethylcellulose

EXAMPLE 8 - Preparation of Debriding Formulation

The debriding enzyme mixture of Example 2 was formulated in the vehicles of EXAMPLES 3 - 7 by manually mixing the lyophilized enzyme powder into the vehicles immediately prior to application. The enzymes were generally formulated at 5% (w/w) for the application described in Example 9, and at 4.5% for the application described in Example 10.

EXAMPLE 9 - In Vitro Assay for Enzyme Delivery Effectiveness

0.5 mL of the debriding formulations described in Examples 3-7 were each extracted with 2.0 mL of 50 mM phosphate buffer pH 6.5. Extraction was carried out by placing the debriding formulation in a 3 cc syringe and connecting the syringe through a 3-way stopcock to a second 3 cc syringe containing the extraction buffer. The buffer was mechanically transferred through the stopcock to the syringe containing the debriding formulation and then transferred back to the parent syringe that originally contained the buffer. This cycle was carried out ten times. The residual vehicle components were removed from the extraction system by micro-centrifugation at 14,000 g for 4 minutes. Approximately 0.4 mL of the aqueous layer was removed from between a resulting pellet and a low density plug at the surface of the microcentrifuge tube using a syringe fitted with a 22 guage needle. The enzyme content of the aqueous layer was assayed using both protein and enzyme activity assays. Protein assays were carried out using the Bradford protein assay (Bio-Rad, Bethesda, MD). Activity assays were carried out using the chromogenic bz-FVR-p-nitroanilide and z-RR-p-nitroanilide protease substrates (Bachem Bioscience Inc., Philadelphia, PA). Stock solutions of the p-nitroanilide substrates were prepared with dimethylformamide as solvent. The assay mix contained the following constituents: 50 mM Hepes buffer, pH 7.5, 5.0 mM L-cysteine, either 0.1 mL of 20 mM bz-FVR-pNA substrate for the assay of ananain or 0.04 mL of z-RR-pNA substrate for the assay of comosain and 0.05 mL of appropriately diluted aqueous phase in a final volume of 1.0 mL. The aqueous phase was diluted 400-fold for the assay with bz-FVR-pNA as substrate and 40-fold for the assay with the z-RR-pNA as substrate.

The results from the extraction assays are reported relative to the amount of enzyme activity and protein extracted from vehicle formulated without thickening agent. These results are shown in Table 2.

Table 2: Debriding Enzyme Extraction Assay

| Vehicle Thickening Agent | Enzyme Units Extracted* | | Protein Extracted* |
|---|---|---|---|
| | bz-FVR-pNA | z-RR-pNA | |
| None | 1.0 | 1.0 | 1.0 |
| Guaran | 0.9 | 0.9 | 0.9 |
| Locust bean gum | 0.9 | 0.9 | 0.9 |
| Xanthan gum | <0.1 | <0.1 | <0.1 |
| Carboxymethyl-cellulose | 0.9 | 0.7 | 0.9 |

*Values are reported relative to that obtained with vehicle formulated without any thickening agent.

These data show both ananain and comosain to be equally extractable with high efficiency from galactomannan-thickened vehicles. Only minimal quantities of the enzymes were extracted from the anionic xanthan gum thickened vehicle and differential quantities of the two enzymes were extractable from the carboxymethylcellulose thickened cream.

EXAMPLE 10 - In Vivo Application of Effectiveness

Twelve 3.5 x 3.5 cm square full-thickness wounds were inflicted on the dorsae of each of four anaesthetized pigs (~50 Kg). The wounds were generated with a 60 gram aluminum block with a 12.25 cm$^2$ exposed face that was heated to 100 °C in an oven. The block was applied to the skin surface of the animals at 1.2 x 10$^4$ Pa for 45 sec. Immediately post burn, the keratin layer was removed from each wound by wiping with a gauze and the wounds were allowed to desiccate approximately 24 h. Prior to treatment, the pigs were once again anaesthetized and 2.2 g of the guaran-thickened debriding formulation was applied to each wound site and the application was covered with an occlusive dressing, Bioclusive™ (Johnson & Johnson, New Brunswick, NJ). After five hours, the formulation was removed, the debrided eschar scraped with a plastic spatula and the partially debrided wound cleansed with a saline-soaked gauze. An additional 2.2 g of debriding formulation was then applied to each wound and debridement allowed to proceed for an additional 5 h period. After 10 hours of treatment, the wounds were cleansed as described for the five hour time point and scored macroscopically for the extent of debridement.

Macroscopic Evaluation:

Macroscopic evaluation was based on two markers; the debridement of melted collagen and the debridement of necrotic cells, such as hair follicles, that lie deeper in the dermis. The following scale was used.

9

| Score | Description of Wound Bed |
|---|---|
| 0 | No debridement |
| 1 | Eschar debridement above hair follicles only |
| 2 | Eschar debridement, some hair removed |
| 3 | Eschar debridement, all hair removed |

Following the 10 hour treatment period, all wounds showed excellent debridement with an average score of 2.66 (N=40). Using this same model, untreated wounds or wounds treated with vehicle alone show scores of 0. These data show the galactomannan-thickened vehicle to be effective at delivering the debriding enzymes.

## Claims

1. A hydrophilic cream for application to an open wound or a mucous membrane characterised in that it comprises an aqueous vehicle, a therapeutic agent selected from debriding enzymes, wound healing agents and antibacterial agents, and a non-pyrogenic seed galactomannan thickening agent.

2. A hydrophilic cream as claimed in claim 1 wherein it is of the oil-in-water type.

3. A hydrophilic cream as claimed in claim 1 or claim 2 wherein the galactomannan is derived from guar gum or locust bean gum.

4. A hydrophilic cream as claimed in any of claims 1 to 3 wherein the galactomannan is guaran.

5. A hydrophilic cream as claimed in any of claims 1 to 4 wherein the debriding enzymes are derived from pineapple stem powder.

6. A hydrophilic cream as claimed in any of claims 1 to 5 wherein the debriding enzymes are ananain and/or comosain.

7. A hydrophilic cream as claimed in any of claims 1 to 6 wherein the open wound is a burn or ulcer.

8. A method for the preparation of a non-pyrogenic seed galactomannan characterised in that it comprises:
   (a) removing insolubles from an aqueous solution of the hydrated galactomannan;
   (b) passing the solution through a cationic membrane to remove the pyrogenic material; and
   (c) precipitating the non-pyrogenic galactomannan from the solution.

9. A method as claimed in claim 8 wherein the galactomannan is guaran.

European Patent
Office

**EUROPEAN SEARCH REPORT**

Application Number

EP    93 30 4941

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 288 945 (HOECHST AKTIENGESELLSCHAFT) | 1,3,4,7 | A61K47/36 |
| Y | * page 2, line 43 - line 53 *<br>* page 3, line 13 - line 16 *<br>* examples 1,3 * | 5,6 | |
| | --- | | |
| D,Y | EP-A-0 313 346 (GENZYME CORPORATION)<br>* claims 1-9 * | 5,6 | |
| | --- | | |
| A | FR-A-2 466 273 (GONZALES, JESUS DIT ALES, PATRICK)<br>* claims 1,3 *<br>* page 2, line 26 - page 3, line 8 *<br>* page 4, line 22 - line 36 * | 2 | |
| | ----- | | |
| | | | **TECHNICAL FIELDS SEARCHED (Int. Cl.5 )**<br><br>A61K |

The present search report has been drawn up for all claims

| Place of search | Date of completion of the search | Examiner |
|---|---|---|
| THE HAGUE | 20 AUGUST 1993 | VENTURA AMAT A. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

............................................................
& : member of the same patent family, corresponding
document

EPO FORM 1503 03.82 (P0401)